# EUROPEAN PATENT SPECIFICATION

(11) **EP 3 442 617 B1**
(45) Date of publication and mention of the grant of the patent: **31.05.2023**
(21) Application number: 17723528.0
(22) Date of filing: 13.04.2017
(51) Int. Cl.: A61M 1/36

(54) **INTRAVASCULAR ACCESS DEVICE DISPLACEMENT ALERTING APPARATUS**
VORRICHTUNG ZUR WARNUNG VOR DER VERSCHIEBUNG EINER INTRAVASKULÄREN ZUGANGSVORRICHTUNG
APPAREIL D'ALERTE DE DÉPLACEMENT DE DISPOSITIF D'ACCÈS INTRAVASCULAIRE

(30) Priority: 13.04.2016 US 201662321900 P
(43) Date of publication of application: 20.02.2019
(73) Proprietor: Fund for Medical Research Development of Infrastructure & Health Services by Barzilai Medical Center, 7830604 Ashkelon (IL); Ashkelon Academic College (Registered Endowment), 7810902 Ashkelon (IL)
(72) Inventor: YAGIL, Yoram, 4640801 Herzlia (IL); LEE, Yuh-Jye, Hsinchu 30010 (TW); KUNG, Po-Yuan, Dounan Township 630 (TW); SHAO, Ya-Lin, New Taipei City 241 (TW); DAVID, Esther, 7856917 Ashkelon (IL); LESHEM, Guy, 7177623 ModiIn (IL)
(74) Representative: RGTH
(86) International application number: PCT/IL2017/050452
(87) International publication number: WO 2017/179060

(56) References cited:
- WO-A1-99/24145
- WO-A1-2008/028594
- WO-A2-2006/120253
- JP-A- 2006 110 119
- US-A1- 2015 374 896

## Description

### FIELD AND BACKGROUND OF THE INVENTION

The present invention relates to apparatuses for an intravascular access device displacement detection. A method is further described, which does not form part of the invention claimed.

Placement of an intravascular access device (IVAD) into the vein or artery of a patient provides critical access to the patient's blood circulation. Displacement of the IVAD from the patient's blood vessel is a constant risk that may result in catastrophic consequences, such as venous or arterial bleeding and excessive blood loss or inadvertent discontinuation of infusion of a life-sustaining drug. Accidental extrusion of the IVAD from its intended position may often go unnoticed, until it is too late.

There is currently no readily available alarm device that can alert the patient or the medical team to the dislodgement of the IVAD.

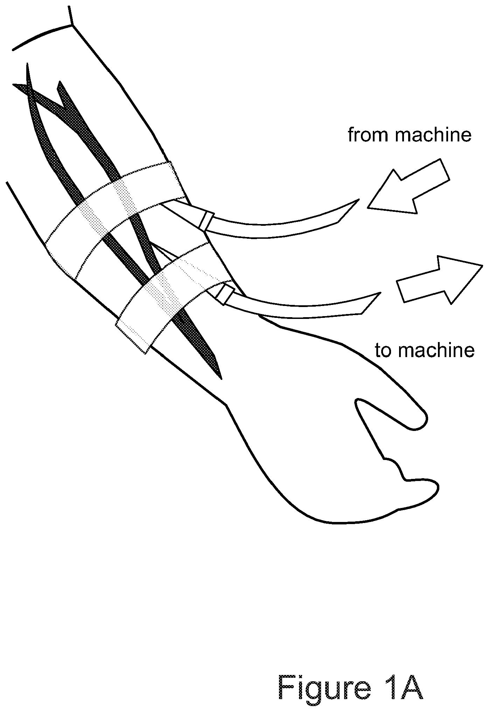

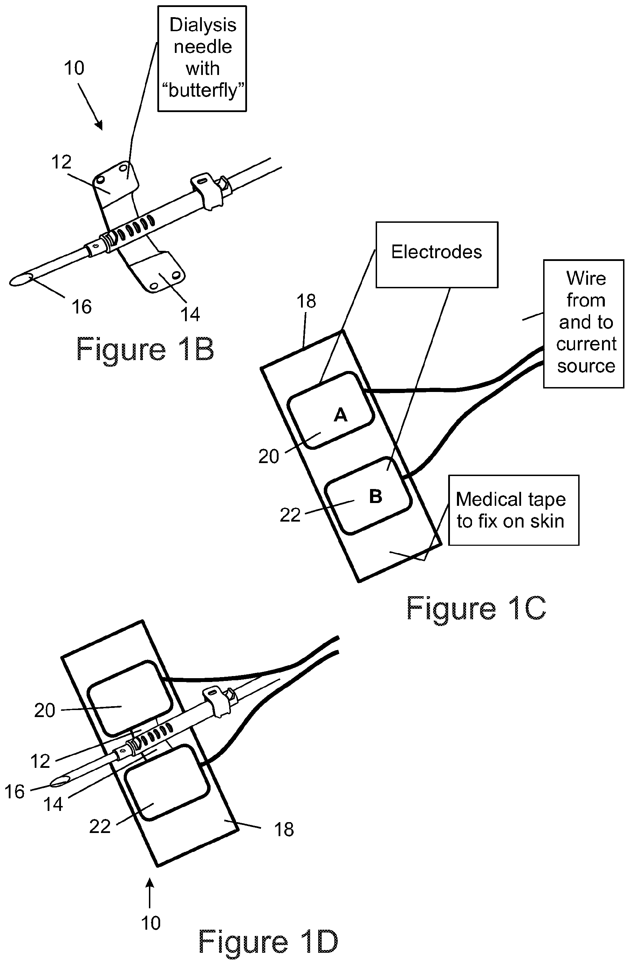

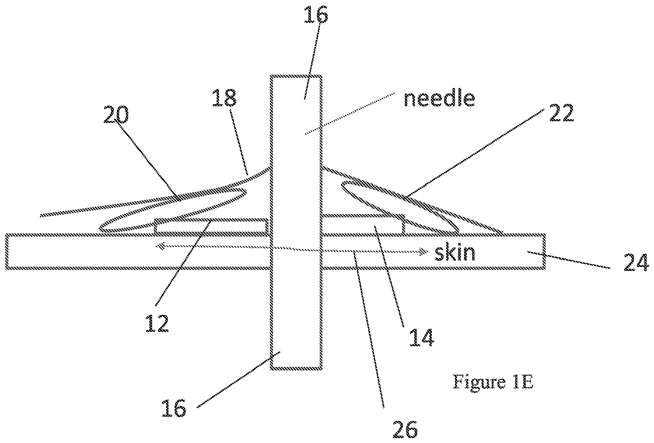

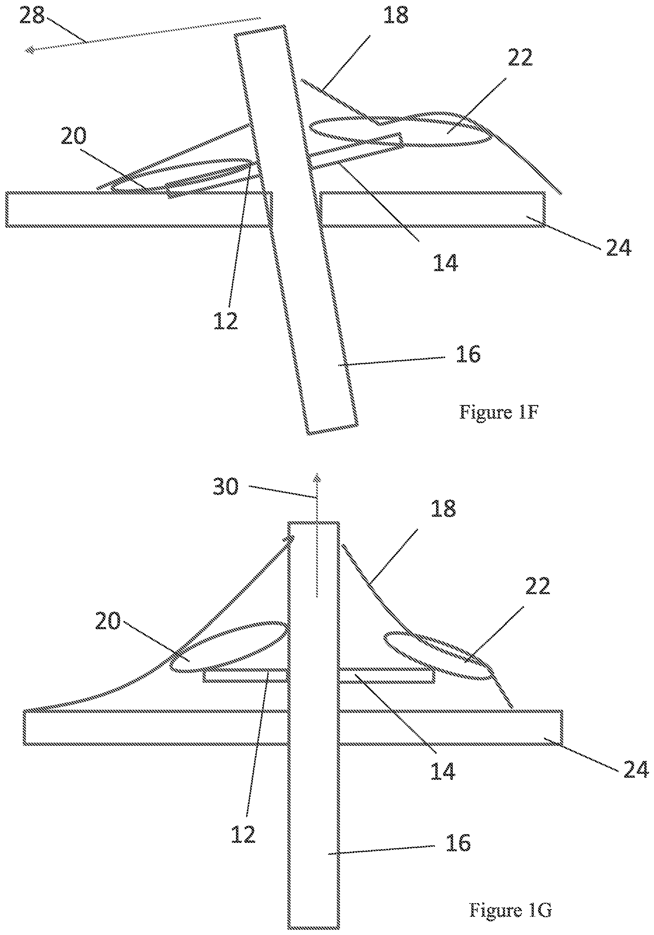

A case in point relates to intravascular needles used in the course of hemodialysis. During hemodialysis, one large gauge needle is inserted into the patient's vascular access (e.g. shunt or fistula) in order to draw blood from the patient (arterial side), and a second large needle is inserted proximally into the shunt in order to return blood that has been purified during the dialysis process to the patient (venous side) (see Fig. 1). During hemodialysis, blood flows through both needles at a relatively high velocity of about 300 to 400 ml/min. Both needles, after being inserted through the skin into the vascular access, are secured and fixed onto the patient's skin, typically with medical tape. Duration of dialysis averages 3-4 hours, during which the patient tends to randomly move the limb with the vascular access. During movement of the limb, one or both access needles are at risk of being inadvertently pulled out of the access, which may result in blood loss and endanger the patient.

Now, when the needle on the arterial side is pulled out inadvertently, the dialysis machine sucks air into the dialysis tubing which is immediately recognized by bubble sensors in the dialysis machine and the dialysis pump automatically and instantly shuts off, simultaneously setting on an alarm. Except for local bleeding, which can be controlled by the nursing team that has been alerted, no further harm comes to the patient. However, if the needle on the venous side is inadvertently pulled out, the results may be catastrophic. Blood continues to be pumped out from the venous side at a fast rate, and is not returned into the patient's circulation but rather flows freely on the patient's clothing or onto the floor, depending on how the needle is displaced. Often the patients cover themselves with a blanket, and the bleeding is underneath the blanket and not timely recognized. Dialysis machines do not at this stage offer an early alarm or fail-safe system for such a scenario. The venous pressure monitored by the dialysis machine eventually drops, but it may take long minutes before an alarm is set off, by which time the patient has lost too much blood, a life-threatening situation.

A second case in point is during plasmapheresis, another condition requiring high blood flow through an extra-corporal machine. Accidental unnoticed dislodging of the intravenous access through which blood is returned to the patient may result in excessive and life endangering blood loss.

A further case in point is during intravenous infusion. The alert system is especially crucial where a life-sustaining drug is intravenously-infused, a non-limiting example may be, a patient in septic shock who is hypotensive is being infused noradrenaline to sustain systemic arterial pressure. Accidental but unnoticed dislodging of the intravenous access may result in interrupted delivery of nor-adrenaline to the patient and catastrophic consequences.

In view of the large variety of health applications as described above, as well as other conditions in which unnoticed dislodgment of an IVAD prevents vital therapy and/or might lead to a disastrous outcome, there is a need for an alert system to timely identify IVAD dislodgment.

Several devices and systems have previously been developed to prevent and/or identify IVAD dislodgment. One example, WO 11/053810 describes an apparatus for detecting disconnection of an IVAD from a blood vessel or vascular graft that comprises: a fluid delivery device for providing fluid through a first conduit into a first site of the blood vessel or graft; a first electrode in contact with the lumen of the first conduit; a second electrode in fluid communication with a second site of the blood vessel or graft; an electronic circuit connected to the first and second electrodes, and configured to deliver a control signal to the first and second electrodes in order to measure the electrical resistance of a fluid between the first and second electrodes.

A second example, WO 03/086504 describes a method for detecting access disconnection during extracorporeal blood treatment, the method comprising the steps of: coupling an extracorporeal blood system including a plurality of components to a patient with an extracorporeal blood circuit including a first blood line and a second blood line such that blood flows into, through and out of the patient along the extracorporeal blood circuit during treatment; injecting an electrical signal into the extracorporeal blood circuit; passing the electrical signal through a conductive connection between the first blood line and the second blood line thereby defining a loop that bypasses one or more components of the extracorporeal blood system coupled to the extracorporeal blood circuit; and measuring a change in an electrical value in response to access disconnection.

A third example, WO 08/100675 describes a system for the detection of access disconnection, which utilizes an electrical circuit with a number of electrical contacts which are in contact with the fluid circuit.

US20150374896 relates to a situation in which bleeding due to the displaced electrodes causes a current to flow between two electrodes.

None of the presently known devices and systems provides a satisfying way of detecting dislodgment of an IVAD from the vein. Moreover, some of the prior art focuses on using electric current passing between two inserted needles and through the patient's blood stream.

JP 2006 110119A discloses an IVAD having electrodes attached to the butterfly wings of the IVAD and which sense touch with the skin. As soon as touch is lost an alert signal is issued.

### SUMMARY OF THE INVENTION

The present embodiments may provide an alerting system that is triggered when the IVAD is displaced from its intended position, and alerts the patient and/or the medical team to the event. The IVAD may be a needle connected for example to an infusion pump or hemodialysis machine, and electrodes having an applied voltage, may be attached to the skin, for example at locations on either side of the needle, in such a way that an electrical micro-current, for example a small or micro-current, flows through the skin between the electrodes. Movement of the needle disrupts contact of the electrodes with the skin and thus disrupts the micro-current, and the absence of the micro-current generates the alert. In this way, in contrast with the prior art, blood does not have to first leak onto the electrodes in order to trigger the alert, and consequently the alert can be that much sooner.

Embodiments may optionally disrupt the action of the associated medical device, so that blood flow is interrupted until the situation is corrected. Thus waste of blood is prevented.

The alert system of the present invention may be applied to any condition that involves placement of an intravascular access device (IVAD) into the vein or artery of a patient for intravenous or intra-arterial use, during which fluids or drugs need to be infused intravenously or intra-arterially, and where accidental unnoticed dislodgement of the vascular access prevents the intended fluid or drug delivery.

According to an aspect of some embodiments of the present invention there is provided an apparatus for intravascular access device displacement detection, said apparatus having the features of claim 1.

In an embodiment, the power source is one member of the group comprising an integral power source and an external power source.

In an embodiment, said alert device is connected to provide said alert signal to said external machine or infusion device.

In an embodiment, said alert device provides said alert signal to a transmitter to provide an external alert.

In an embodiment, said external alert is one member of the group consisting of a tactile alert, an audio alert, a visual alert and a combination thereof.

In an embodiment, said alert signal is configured to notify the patient or his caregivers of displacement detection.

In an embodiment, said external alert is at a remote location.

In an embodiment, the intravascular access device comprises butterfly wings for holding said needle to the skin at said access site and said electrodes are configured to be attached over the butterfly wings and to the skin beyond edges of the butterfly wings.

In an embodiment, the intravascular access device is configured to be attached to the skin at the access site using tape and the electrodes are configured to be attached at the access site with respect to the intravascular access device.

In an embodiment, said electrodes are attached to said tape, either when attaching the device or they may be pre-attached to the tape.

In an embodiment, said electrodes are independently attached to the access site with respect to the intravascular access device.

In an embodiment, said electrodes are secured to said intravascular access device such that displacement of said intravascular access device causes displacement of one or both of said electrodes from said skin.

According to a second aspect of the present invention there is provided an apparatus with the features of claim 11.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method (which does not form part of the invention claimed) and/or system of embodiments of the alert system of the present invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method (not claimed) and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRA WING(S)

Some embodiments of the invention are herein described, by way of example only, with reference to the accompanying figures. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figure 1A is a simplified drawing showing IVADs secured on an arm;
Figure 1B is a simplified view of a butterfly needle;
Figure 1C is a simplified view of electrodes on a surgical tape designed to hold a butterfly needle on the skin according to embodiments of the present invention;
Figure 1D is a simplified diagram showing the tape of Fig. 1C connected across the needle and butterfly wings of Fig. 1B to provide displacement detection according to embodiments of the present invention;
Figure 1E is a cross-sectional view from the front of the needle of FIG. 1C secured by the surgical tape across the butterfly wings and the electrodes being connected under the tape and across the wings to the patient's skin so as to be mobile with the tape and wings;
Figure 1F is a simplified diagram showing in cross section a needle being displaced to one side and showing how the electrodes are displaced from the skin, allowing for detection of the displacement, according to an embodiment of the present invention;
Figure 1G is a simplified diagram showing in cross section a needle being displaced upwardly and showing how the electrodes are displaced from the skin, allowing for detection of the displacement, according to an embodiment of the present invention;
Figure 2A is a simplified block diagram showing parts of a displacement alert unit according to an embodiment of the present invention;
Figure 2B is a simplified block diagram showing a variation of the embodiment of FIG. 2A with an internal power source;
Figures 3A to 3C show three different examples of IVADs to which the present embodiments may be applied;
Figure 4A to 4D illustrate four exemplary constructions in which electrodes are applied to an IVAD;
Figure 5 is a simplified diagram in which IVADs with displacement detectors are applied to the leg of a patient;
Figure 6 is a simplified diagram showing an alternative IVAD with a butterfly needle;
Figure 7 is a simplified diagram showing a method (not forming part of the invention claimed) of inserting an IVAD with a displacement detection system according to embodiments of the present invention; and
Figures 8A and 8B are two parts of an example of an IVAD with displacement detection according to embodiments of the present invention.

### DESCRIPTION OF SPECIFIC EMBODIMENTS OF THE INVENTION

The invention is defined by the appended claims and relates to a displacement detector for an intravascular access device and, more particularly, but not exclusively, to a detector for indicating situations where displacement may lead to loss of blood.

Displacement of intravascular access devices (IVAD), such as a needle or a catheter, is a known risk in all procedures that involve IVAD insertion to a patient, especially in prolonged procedures such as renal replacement therapy (hemodialysis, hemofiltration, hemodiafiltration) or during intravenous infusion or during the need for direct intra-arterial access. IVAD displacement may result in a variety of outcomes, ranging from loss of fluids onto the floor instead of being administered to the patient, to discontinuation of a life-sustaining medication, or fatal loss of blood that is not returned to the patient's body after extraction for purification, e.g. during hemodialysis or plasmapheresis. Accordingly, a need exists for an alerting device that sets on an alarm, notifies the healthcare provider of such IVAD displacement, and generates a signal that interrupts any associated electrical or mechanical medical device

An apparatus for intravascular access device displacement detection is described, where an intravascular access device is inserted into a blood vessel through the skin at an access site. The apparatus comprises electrodes located on either side of the intravascular access device which electrodes are associated with the intravascular access device and are attachable to the skin about the access site. By "about" is meant in the vicinity of or "in close proximity to". The term "electrodes" refers to two separated conductive surfaces of any size which allow an electric micro-current to flow between them through the patient's skin. The electrodes have micro-current flowing there between, and through the skin between the electrodes and the micro-current persists as long as the electrodes are connected to the skin. If the intravascular access device moves then one or both of the electrodes is disconnected and the micro-current is interrupted. A displacement alert device issues an alert signal upon degradation or cessation of the induced micro-current.

The intravascular access device is secured to the user's skin so that the needle is securely held in place. Securing may be via an adhesive tape, and the electrodes may be attached to the tape or may be otherwise connected so as to be mobile with the intravascular device.

More particularly, the present embodiments may provide a micro-current through the user's skin between the electrodes mounted in such a way with respect to the intravascular access device needle, that movement of the needle pulls one or both of the electrodes away from contact with the skin. The electrodes may for example be mounted on the tape that attaches the needle to the patient, as described above. In an embodiment the electrodes may be attached to the needle so that when the needle is taped to the skin, the electrodes are trapped between the tape and the skin. As long as the needle is in position the electrodes remain in their locations on the skin and the electrical current remains intact. As the needle is displaced the electrical circuit through the user's skin is broken and that cessation of the current between the two electrodes may be used as an indication that the needle has been displaced. The electrodes may be positioned far enough apart that they do not touch each other and short when displaced, yet may be close enough together that a measurable micro-current passes between the electrodes when attached to the skin. In an embodiment the electrodes are attached at two opposite sides of the needle.

That is to say, there may be a current between the electrodes or there may not be. The electrodes become detached from the skin due to displacement of the IVAD device, thus causing the previously existing current flow through the skin to stop.

The electrodes are connected to a power source, and the power source may be internal or external, to provide a voltage between the electrodes and thus set up the electric current. The disruption of the electrical circuit may be used as a signal to set off an alarm or like output.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Referring now to the drawings, Figure 1A illustrates the arm of a patient with two IVADs, one drawing blood from the body and one returning blood to the body. It will be appreciated that in the case of infusion there is only one IVAD.

Referring now to Fig. 1B and there is shown a butterfly needle 10 which is typically used for dialysis or to administer substances to the body and the like. The butterfly arms 12 and 14 are typically secured to the skin by medical tape so that the needle 16 does not move in use.

As shown in Fig. 1C medical tape 18 used to secure the butterfly needle 10 has electrodes 20, 22 emplaced therein. The electrodes are connected to a power supply, which provides the micro-current circuit therebetween.

Referring now to Fig. 1D the tape 18 is placed over the butterfly wings and on the skin around the wings, with the electrodes 20 and 22 bridging between the surfaces of the butterfly wings and the skin of the patient. An electrical path is established through the skin and a current, typically a small current, or micro-current, passes through the patient's skin between the two electrodes. The term 'micro-current' may include any current most effectively measured in microamperes, and whose passage may not feel uncomfortable to the patient. In certain embodiments, the micro-current being used is less than 100 microampere; less than 10 microampere; or less than 1 microampere.

A slight movement of either of the butterfly wings may disturb the corresponding electrode, so that one of the electrodes, or both, ceases to be in contact with the skin. Thus the micro-current is interrupted.

Reference is now made to Fig. 1E which is a cross-section from the front of the arrangement of Fig. 1D. Needle 16 penetrates the skin 24, and is held in place by butterfly wings 14 and 16. Tape 18 holds the needle in place over the butterfly wings and the electrodes 20 and 22 are sandwiched between the butterfly wings and the tape so as to contact the skin outside the butterfly wings. Arrow 26 indicates the electrical micro-current through the skin between the outer edges of the electrodes extending over the ends of the butterfly wings.

Reference is now made to Fig. 1E, which is a cross-section illustrating what happens when the butterfly needle 16 of Fig. 1A is pulled to one side, in the direction of arrow 28. One of the two butterfly wings 16 is pulled up and away from the skin, pulling out the corresponding electrode 22. Butterfly wing 14 and corresponding electrode 14 remain in position, however the micro-current is interrupted.

Reference is now made to Fig. 1F, which illustrates what happens when needle 16 is displaced upwards in the direction of arrow 30. Both butterfly wings 12 and 14 are pulled away from the skin, pulling the electrodes 20 and 22 with them. Thus the electrodes are pulled away from the skin and, again, the electrical circuit through the skin is broken.

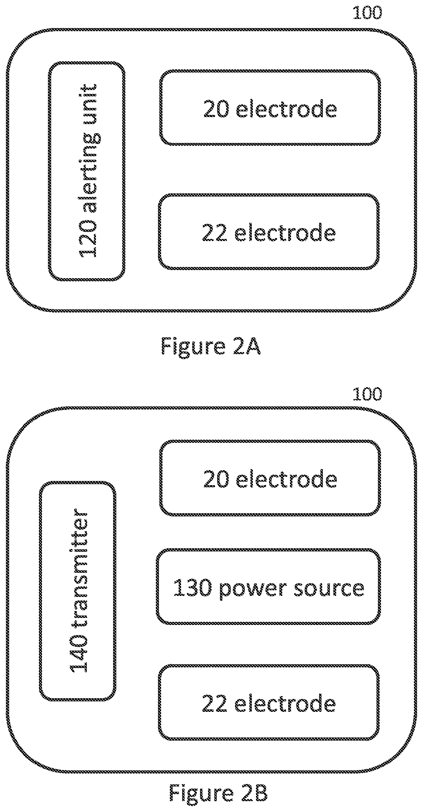

Reference is now made to Fig. 2A, which is a block diagram of an IVAD displacement alerting device 100 according to the present embodiments which may detect and provide an alarm, alert or warning regarding accidental displacement of the needle 16 or IVAD 10 as a whole. The device 100 may comprise: (a) electrodes 20 and 22 associated with the IVAD 16 as explained above; (b) an alerting unit 120; and (c) a power source 130, which may be internal or external, but in either case allows a voltage to appear across the electrodes which is sufficient to establish the micro-current when the electrodes are in position on the patient's skin. When inserting the needle 16 into the patient's veins or arteries, and fixing the needle in place, the electrodes 20 and 22 are brought into contact with the patient's skin 24 around the site of the IVAD 10 insertion, e.g. of the infusion catheter or needle 16. As a result, a current flows through the user's skin between the electrodes, but as soon as the needle is displaced, one or both of the electrodes may cease to be in contact with the skin, as explained, and the current path is disrupted. Thus, as long as the electrodes 20 and 22 are in position, a micro-current, produced by the integral power source 130 or an external power source, is able to run between the electrodes 20 and 22 through the patient's skin, and upon displacement of the IVAD 10, when one or both of the electrodes 20, 22 ceases to touch the patient's skin, the movement interrupts the micro-current run and activates alerting system 120.

The IVAD displacement alerting device 100 may comprise electrodes 110 and an alerting system 120. The device may further comprise, or be connected externally to, a power source. The device may set off a local alarm or may transmit a signal in order to set off an external alert. An external alert may be a remote alarm, and the term may further include a signal that is sent to a machine. For example a signal may be sent that is able to interact with the external machine and for example cause parts of the external machine such as a pump in order to shut off fluid pumping, close valves or perform other actions to protect the patient. In order to transmit the signal a transmitter 140 may be provided to contact an externally located alerting unit 120 as shown in Fig. 2B, and may include or be connected to a computing device, a display unit, a visual alert, an audio alert, etc. or any combination thereof. The computing device may be any suitable device, including a mobile telephone or tablet or laptop or desktop computer or the like.

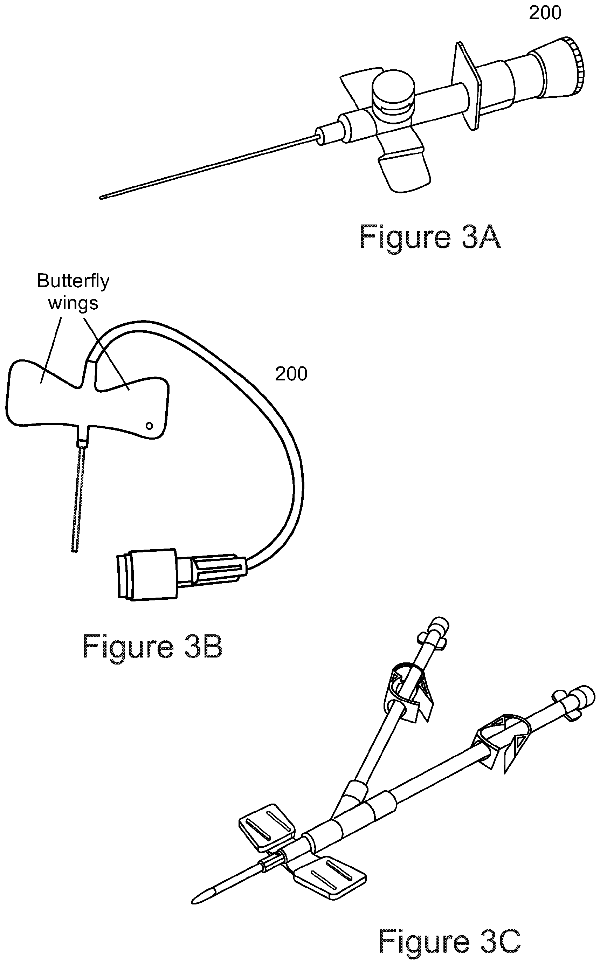

Reference is now made to Figs 3A to 3C which show three different examples of the IVAD 10. In certain embodiments, the IVAD 10 may comprise an infusion catheter as shown in Fig. 3A, a butterfly needle as shown in Fig. 3B, or a double-branched needle as shown in Fig. 3C.

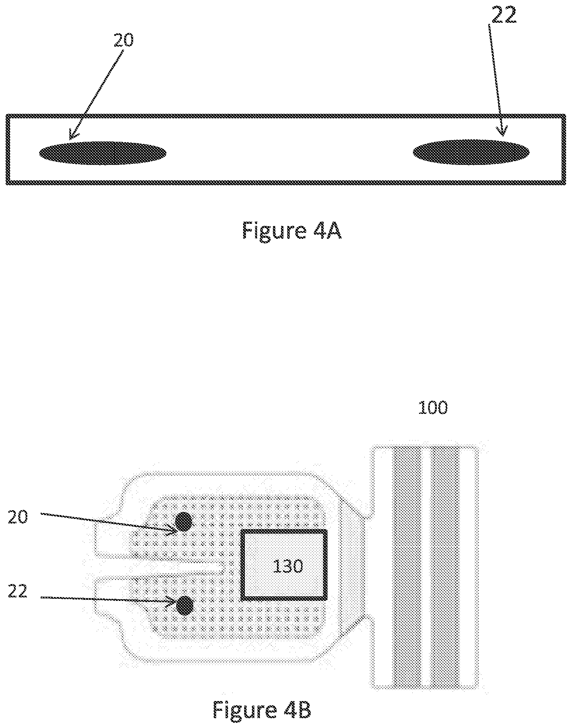

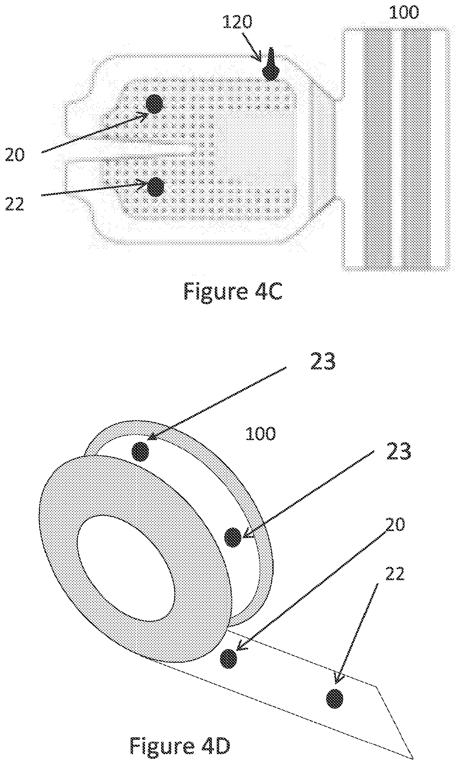

Reference is now made to Figs 4A to 4D, which shown different embodiments in which the IVAD displacement alerting device **100** is incorporated into an adhesive tape, such that when using the tape to affix an IVAD in place after insertion into the patient's body, the electrodes **20** and **22** become mobile with the IVAD, as opposed to being mobile independently, and are brought into contact with the patient's skin. Examples of an adhesive tape include, for example, simple adhesive stripe (Fig. 4A), dedicated tape for catheters (Figs. 4B and 4C), or a roll of adhesive tape (Fig. 4D). In a specific embodiment, the roll of adhesive tape contains numerous electrodes 23 spaced at suitable intervals, is designed so that the user can cut the tape according to need, such that at least two electrodes **20, 22** are held in the piece of cut tape.

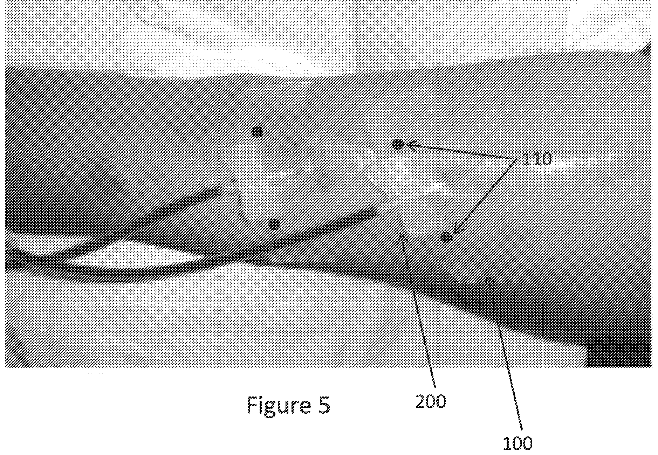

As seen in Fig. 5, an IVAD displacement alert device **100** of the present embodiments is embedded within an adhesive tape. The tape is placed across the needle and butterfly wings to attach an IVAD to the patient's body in a simple and fast manner so that the electrodes attach to the skin at or near the wing tips. The procedure is intuitive for medical staff who already know how to tape butterfly needles to the skin and does not require any special procedure, training or preparation.

In certain embodiments, an IVAD displacement alerting system may comprise (a) electrodes **110** connected-to or associated-with the IVAD **10;** (b) a power source **130;** (c) a transmitter **140;** and (d) an alert unit **120** coupled to the electrodes **110** and to the power source **130,** and adapted for producing an alert upon the displacement of the IVAD. In a specific embodiment, the displacement is identified by simply detecting that the micro-current passing between the electrodes **110** is interrupted.

In certain embodiments, an IVAD displacement alerting system may comprise: (a) electrodes **110** connected to or associated-with the IVAD **200;** (b) a power source **130;** (c) a transmitter **140;** (d) a computer comprising a processor and a memory, coupled to the electrodes **110,** and optional transmitter **140,** the computer being adapted to receive data from the electrodes **110** and/or from the optional transmitter **140,** and designed to analyze the data; and (e) an alert unit **120** coupled to the computer, and adapted for producing an alert if the analyzed data is determined to indicate IVAD displacement.

In a specific embodiment, an IVAD **10** includes any one of the IVAD displacement alerting devices **100** as described above.

In the above, both electrodes are shown to be attached to the tape. In an alternative embodiment, IVAD displacement alerting device **100** itself contains one of the electrodes **20,** and the IVAD **10,** to be inserted into a blood vessel of the patient, comprises another electrode **22,** thereby enabling micro-current to pass in between the two electrodes **20 and 22** as before.

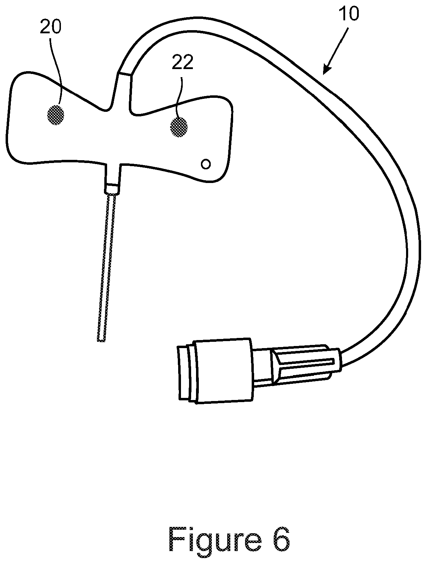

Reference is now made to Fig. 6, which is a simplified diagram showing an embodiment in which the IVAD **200** itself includes electrodes **20** and **22.** Other components of the IVAD displacement alerting device **100** may likewise be built into the IVAD, such as power source **130,** transmitter **140,** a computer comprising a processor and a memory, coupled to the electrodes **110,** transmitter **140** and alert unit **120** coupled to the computer.

As also discussed elsewhere above, the term "electrode" as used herein may refer to two separate conductive surfaces of any size which allow an electronic circuit to run through the skin and between the two conductive surfaces. The conductive surfaces may be any electrical conductive material suitable for introducing a voltage across the skin in order to allow the flow of micro-current.

In certain embodiments, the electrodes **110** are made of any suitable material, such as silver, gold, gold-plated material, titanium, etc.

In certain embodiments, the IVAD displacement alerting device **100** may include a transmitter **140,** wherein the alerting unit **120** or a parallel alerting unit, is located at a remote location, e.g., at the nursing team's desk / computer / smartphone, such that the transmitter **140** sends an alert to the remote alerting unit. The transmitter **140** can send signals to the alerting unit in any suitable way, such as by radiofrequency (RF), infrared (IR), Bluetooth, Wi-Fi, etc.

In certain embodiments, the power source being used to activate the IVAD displacement alerting device **100** of the invention, i.e., creating the micro-current between the electrodes **110** and sending the alert, is an integral part of the device **100,** e.g. a battery, or is an external battery, e.g. placed onto or near the IVAD **200.** Alternatively, the power source is an external power source, such as the main power grid or alternatively from a machine connected to the alerting device **100,** e.g. a dialysis machine. The power source, whether integral or external, is connected to provide the micro-current to run between the electrodes **110,** to activate the alerting unit **120,** and to supply power to any other component of the alerting device **100** or alerting system that needs electric power, e.g., the transmitter **140.**

Accordingly, in certain embodiments, the IVAD displacement alerting device **100** of the present embodiments further comprises a computer having a processor and a memory, such that the computer monitors the micro-current flow between the electrodes **100** and sends out an alert through the alert unit **120** upon interruption thereof, which occurs during an IVAD displacement.

In certain embodiments, the term "computer" as used herein refers to any type of computer, including, but not limited to, a desktop computer, a laptop computer, a smart phone, or a tablet.

In a specific embodiment, the IVAD displacement alerting device may comprise: (a) a sensor associated with the IVAD **10;** (b) an alarm unit; and (c) optionally, a power source, wherein the sensors are in contact with the patient's skin at the site of IVAD insertion, thereby allowing constant measuring, such that upon displacement of the IVAD from the patient's skin the sensor fails to touch the skin, thereby interrupting or altering the measurements and activating the alarm unit. In a specific embodiment, the IVAD displacement alerting device comprises one, two, three, or more than four sensors, wherein the sensors are designed to measure the same or different parameters, such as: electricity, conductivity, distance (from the patient's skin), temperature, perspiration. etc., or any combination thereof. In a specific embodiment, the device further comprises a computer having a processor and a memory, designed to monitor and analyze the data measured by the sensors, and to send an alert through the alert unit upon identification of changes that are indicative of IVAD displacement, e.g., increased distance, decrease in measured perspiration or temperature, etc.

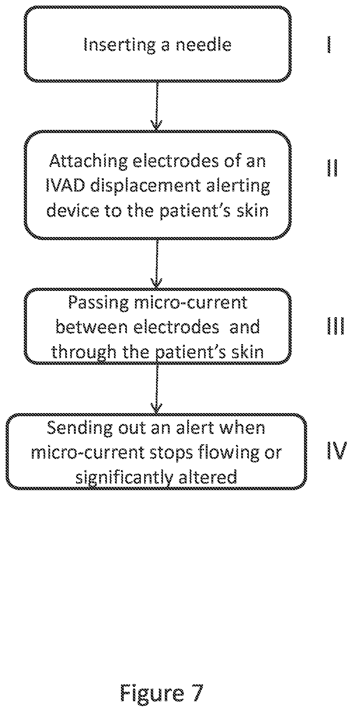

Reference is now made to Fig. 7, which is a simplified flow chart illustrating a method for detecting an IVAD **200** displacement according to the present embodiments. The method comprises: (a) attaching the electrodes **20, 22** of an IVAD displacement alerting device **100** of the present embodiments to the patient's skin in proximity to the IVAD **10** insertion area, while associating the device **100** with the IVAD **10,** so that the electrodes are mobile with the IVAD rather than independently; (b) initiating a micro-current to run between the electrodes **20, 22,** thereby creating a reference condition; and (c) when the micro-current is interrupted, determining that IVAD displacement has occurred. Upon determination of IVAD displacement (e) alerting the user or health provider of the occurrence.

Fig. 7 illustrates one possible way of performing the IVAD displacement alerting technique / method according to the invention: after inserting the IVAD **200** into the patient's body (I) and while affixing it in place (or soon after), the electrodes **110** of the IVAD displacement alerting device **100** of the invention are attached to the patient's skin (II); then, the device **100** is activated and a micro-current is passed between the electrodes **110** through the patient's skin (III); and as long as the micro-current flows without interference, meaning that the IVAD **200** is in place, the alerting unit **120** is silent. However, once the IVAD **200** is displaced, at least one of the electrodes **20, 22** is disconnected from the patient's skin, which results in the termination or weakening or like noticeable alteration of the micro-current flow, the alarm unit **120** is activated and an alarm is set (IV). It should be noted that this scenario is merely an example, and the skilled artisan will apply various modifications according to specific needs or circumstances including specific designs of the IVAD displacement alerting device **100.**

In a specific embodiment of the method, which does not form part of the invention claimed, the IVAD displacement alerting device **100** is attached-to or associated-with the IVAD **10** by any suitable means, such as, but not limited to, glue, welding, surface attachment, adhesive tape, or fasteners; or is an integral part of the IVAD **10** and is manufactured therewith. In a specific embodiment, the IVAD displacement alerting device **100** is fabricated as part of an adhesive tape used to affix the IVAD **10** in place.

In an embodiment, once the IVAD displacement alerting device **100** or alerting system has determined that the IVAD **10** has been displaced, in addition to the alarm being activated, the alerting device **100** or alerting system may actively and automatically deactivate the associated machine, e.g. the hemodialysis machine or the infusion machine, to thereby prevent blood loss or loss of infusion material / medications even before the arrival of the medical team. Such deactivation may include deactivation of pumps or closing of valves.

### EXAMPLES

### Example 1

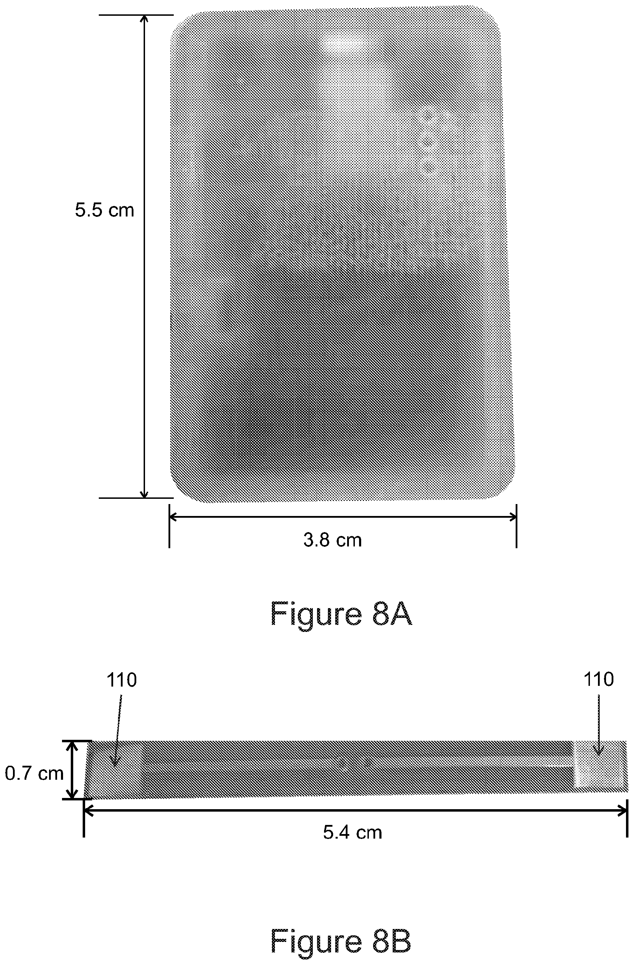

As illustrated in Figs. 8A and 8B, in a specific embodiment the IVAD displacement alerting device **100** according to the invention comprises two separate units: a "dongle" comprising a power source **130** and an alerting unit **120** (Fig. 8A); and electrodes **20, 22.** The dongle and the electrodes are connected by wire.

The power source within the dongle is configured to provide long shelf life; a standby time of at least about 24 hours; and an operation time of at least about 4 hours. It should be noted that the above mentioned periods are susceptible to change according to the battery being used and the storing conditions, and can be extended as needed, e.g. according to the duration of the medical procedure the patient undergoes.

As illustrated in Fig. 8A, the size of the exemplified dongle is about 5.5 ^{∗} 3.8 ^{∗} 2 cm³. However, as can be easily understood by a skilled artisan, these parameters can be modified and can vary. For instance, the length and width of the dongle can be up to 10 cm or more, or less than 2 cm, and the thickness may be from 0.3 to 5 cm.

As illustrated in Fig. 8B, the size of the exemplified electrodes **20, 22** is about 0.7 * 5.4 cm. However, as can be easily understood by a skilled artisan, these parameters may be modified and/or adjusted as needed, e.g. according to the size of the IVAD or butterfly IVAD being used. For instance, the length of the electrodes can be from about 0.5 cm to about 15 cm or more, and the width may be from about 0.1 to about 2 cm. The distance between the two electrodes in the exemplified electrodes is about 4.5 cm. However, this distance is exemplary only and the skilled artisan may make modifications to fit the given circumstances. For instance, the distance between the electrodes may vary from about 0.2 cm to about 15 cm.

The electrodes may be made from any suitable material known in the art. In the present example, the electrodes are made of flexible copper clad laminate (FCCL), having 3 layers: copper clad; epoxy; and polyester. The FCCL in the present example has an electro deposited copper resistivity of 1.67Ω·m and permittivity of 3.0 @ 1MHz.

It is expected that during the life of a patent maturing from this application many relevant IVAD technologies will be developed and the scope of the term IVAD is intended to include all such new technologies *a priori.*

The terms "comprises", "comprising", "includes", "including", "having" and their conjugates mean "including but not limited to".

As used herein, the singular form "a", "an" and "the" include plural references unless the context clearly dictates otherwise.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims

## Claims

1. An apparatus for intravascular access device displacement detection, the intravascular access device (10) for insertion into a blood vessel through the skin at an access site and to be attached thereto, the intravascular access device (10) being connected to an external machine or infusion device, the apparatus comprising:
electrodes (20, 22) for attachment to the skin at the access site and to be located at first and second sides respectively of said intravascular access device (10), the electrodes (20, 22) being connected to a power source (130), the power source providing a measurable micro-current, **characterized by** the electrodes (20, 22) allowing said measurable micro-current to flow therebetween through the patient's skin;
the apparatus further **characterized by** an alert device (120) associated with said electrodes (20, 22) and to issue an alert signal upon interruption of said micro-current flowing between said electrodes (20, 22), said alert signal being configured to shut down a fluid supply of said external machine or infusion device to said intravascular access device (10); and
the apparatus further being **characterized by** a dongle holding said power source (130) and said alert device (120).

2. The apparatus according to claim 1, wherein said alert device (120) is connected to provide said alert signal to said external machine or infusion device.

3. Apparatus according to claims 1 or 2, wherein said alert device (120) provides said alert signal to a transmitter to provide an external alert.

4. The apparatus according to claim 3, wherein said external alert is one member of the group consisting of a tactile alert, an audio alert, a visual alert and a combination thereof.

5. The apparatus according to claim 3, wherein said alert signal is configured to notify the patient or his caregivers of displacement detection.

6. The apparatus according to claim 3, wherein said external alert is at a remote location.

7. The apparatus according to any one of the preceding claims, wherein the intravascular access device (10) comprises butterfly wings for holding said needle to the skin at said access site and said electrodes (20, 22) are configured to be attached over the butterfly wings and to the skin beyond edges of the butterfly wings.

8. The apparatus according to any one of the preceding claims, wherein the intravascular access device (10) is configured to be attached to the skin at the access site using tape and the electrodes (20, 22) are configured to be attached at the access site with respect to the intravascular access device (10).

9. The apparatus according to claim 8, wherein said electrodes (20, 22) are - attached to said tape.

10. The apparatus according to any one of the preceding claims, wherein said electrodes (20, 22) are independently attached to the access site with respect to the intravascular access device (10), or wherein said electrodes (20, 22) are secured to said intravascular access device (10) such that displacement of said intravascular access device causes displacement of one or both of said electrodes (20, 22) from said skin.

11. Apparatus for providing a displacement alert for an intravascular access device (10) inserted into a patient's blood vessel through the skin at an access site, the apparatus comprising:
electrodes (20, 22) for attachment to said skin across said access site, , the electrodes (20, 22) comprising flexible copper clad laminate (FCCL), having 3 layers: copper cladding, epoxy, and polyester;
a power source connected to said electrodes (20, 22);
said electrodes (20, 22) being associated with said intravascular access device (10) such that at least one of said electrodes (20, 22) becomes detached from said skin upon displacement of said intravascular access device (10), thereby disrupting said micro-current;
a transmitter for transmitting an alarm signal upon interruption of micro-current circuit; and
a receiver for receiving said alarm signal and for producing a displacement alert output, **characterized in that**:
said electrodes are configured to facilitate a measurable micro-current through said skin and between said electrodes (20, 22) across said access site, the power source being located in a dongle including an alert device (120),
the power source providing said measurable micro-current to said electrodes, the micro-current flowing from a first of said electrodes (20, 22) through the patient's skin to a second of said electrodes (20, 22).

12. The apparatus of claim 11, wherein said displacement alert output is configured to operate a fluid handling machine handling fluid taken from or being provided to said intravascular access device (10) to put said fluid handling machine in a safety state, or is configured to operate a remote alarm.

## Patentansprüche

1. Gerät zur Erkennung einer Verschiebung einer intravaskulären Zugangsvorrichtung, wobei die intravaskuläre Zugangsvorrichtung (10) zum Einführen in ein Blutgefäß durch die Haut an einer Zugangsstelle und zur Befestigung an dieser vorgesehen ist , wobei die intravaskuläre Zugangsvorrichtung (10) mit einer externen Maschine oder Infusionsvorrichtung verbunden ist und das Gerät Folgendes umfasst:
Elektroden (20, 22) zum Befestigen an der Haut an der Zugangsstelle, und zum Anordnen an einer ersten bzw. zweiten Seite der intravaskulären Zugangsvorrichtung (10), wobei die Elektroden (20, 22) mit einer Stromquelle (130) verbunden sind, wobei die Stromquelle einen messbaren Mikrostrom bereitstellt, **dadurch gekennzeichnet, dass** die Elektroden (20, 22) es dem messbaren Mikrostrom ermöglichen, dazwischen durch die Haut des Patienten zu fließen,
das Gerät ferner **gekennzeichnet ist durch** eine Alarmvorrichtung (120), die den Elektroden (20, 22) zugeordnet ist, und dazu ausgebildet ist, bei Unterbrechung des zwischen den Elektroden (20, 22) fließenden Mikrostroms ein Alarmsignal auszugeben, wobei das Alarmsignal so konfiguriert ist, dass es eine Fluidzufuhr der externen Maschine oder Infusionsvorrichtung zu der intravaskulären Zugangsvorrichtung (10) abschaltet; und
das Gerät ferner **gekennzeichnet ist durch** einen Dongle, der die Stromquelle (130) und die Alarmvorrichtung (120) enthält.

2. Gerät gemäß Anspruch 1, wobei die Alarmvorrichtung (120) verbunden ist, um der externen Maschine oder Infusionsvorrichtung das Alarmsignal bereitzustellen.

3. Gerät gemäß Anspruch 1 oder 2, wobei die Alarmvorrichtung (120) das Alarmsignal einem Sender bereitstellt, um einen externen Alarm bereitzustellen.

4. Gerät gemäß Anspruch 3, wobei der externe Alarm ein Element der Gruppe ist, die aus einem taktilen Alarm, einem akustischen Alarm, einem visuellen Alarm und einer Kombination davon besteht.

5. Gerät gemäß nach Anspruch 3, wobei das Alarmsignal so konfiguriert ist, dass es den Patienten oder seine Betreuer über eine Erkennung einer Verschiebung benachrichtigt.

6. Gerät gemäß Anspruch 3, wobei sich der externe Alarm an einem entfernten Ort befindet.

7. Gerät gemäß einem der vorhergehenden Ansprüche, wobei die intravaskuläre Zugangsvorrichtung (10) Schmetterlingsflügel zum Halten der Nadel an der Haut an der Zugangsstelle umfasst und die Elektroden (20, 22) so konfiguriert sind, dass sie über den Schmetterlingsflügeln und an der Haut jenseits der Ränder der Schmetterlingsflügel befestigt werden.

8. Gerät gemäß einem der vorhergehenden Ansprüche, wobei die intravaskuläre Zugangsvorrichtung (10) so konfiguriert ist, dass sie an der Zugangsstelle unter Verwendung von Klebeband an der Haut befestigt wird, und die Elektroden (20, 22) so konfiguriert sind, dass sie an der Zugangsstelle in Bezug auf die intravaskuläre Zugangsvorrichtung (10) befestigt werden.

9. Gerät gemäß Anspruch 8, wobei die Elektroden (20, 22) an dem Klebeband befestigt sind.

10. Gerät gemäß einem der vorhergehenden Ansprüche, wobei die Elektroden (20, 22) unabhängig an der Zugangsstelle in Bezug auf die intravaskuläre Zugangsvorrichtung (10) befestigt sind, oder wobei die Elektroden (20, 22) an der intravaskulären Zugangsvorrichtung (10) so gesichert sind, dass eine Verschiebung der intravaskulären Zugangsvorrichtung eine Verschiebung einer oder beider Elektroden (20, 22) von der Haut bewirkt.

11. Gerät zum Bereitstellen eines Verschiebungsalarms für eine intravaskuläre Zugangsvorrichtung (10), die an einer Zugangsstelle durch die Haut in ein Blutgefäß eines Patienten eingeführt wird, wobei das Gerät Folgendes umfasst:
Elektroden (20, 22) zur Befestigung an der Haut über die Zugangsstelle, wobei die Elektroden (20, 22) ein flexibles kupferkaschiertes Laminat (FCCL) umfassen, das 3 Schichten aufweist: Kupferkaschierung, Epoxidharz und Polyester;
eine Stromquelle, die mit den Elektroden (20, 22) verbunden ist,
wobei die Elektroden (20, 22) mit der intravaskulären Zugangsvorrichtung (10) verbunden sind, so dass mindestens eine der Elektroden (20, 22) beim Verschieben der intravaskulären Zugangsvorrichtung (10) von der Haut gelöst wird, wodurch der Mikrostrom unterbrochen wird;
einen Sender zum Senden eines Alarmsignals bei Unterbrechung des Mikrostromkreises;
einen Empfänger zum Empfangen des Alarmsignals und zum Erzeugen einer Verschiebungsalarmausgabe,
**dadurch gekennzeichnet, dass**
die Elektroden so konfiguriert sind, dass sie einen messbaren Mikrostrom durch die Haut und zwischen den Elektroden (20, 22) quer durch die Zugangsstelle erleichtern, wobei sich die Stromquelle in einem Dongle einschließlich einer Alarmvorrichtung (120) befindet,
die Stromquelle den messbaren Mikrostrom an die Elektroden bereitstellt, wobei der Mikrostrom von einer ersten der Elektroden (20, 22) durch die Haut des Patienten zu einer zweiten der Elektroden (20, 22) fließt.

12. Gerät gemäß Anspruch 11, wobei die Verschiebungswarnausgabe so konfiguriert ist, dass sie eine Flüssigkeitsbearbeitungsmaschine betreibt, die Flüssigkeit bearbeitet, die von der intravaskulären Zugangsvorrichtung (10) entnommen oder ihr zugeführt wird, um die Flüssigkeitsbearbeitungsmaschine in einen Sicherheitszustand zu versetzen, oder so konfiguriert ist, dass sie einen Fernalarm bewirkt.

## Revendications

1. Appareillage pour la détection de déplacement d'un dispositif d'accès intravasculaire, le dispositif d'accès intravasculaire (10) étant destiné à l'insertion dans un vaisseau sanguin à travers la peau sur un site d'accès et destiné à être fixé sur celui-ci, le dispositif d'accès intravasculaire (10) étant relié à une machine externe ou un dispositif de perfusion, l'appareillage comprenant :
des électrodes (20, 22) destinées à être fixées sur la peau sur le site d'accès et à être situées à des premier et second côtés respectivement dudit dispositif d'accès intravasculaire (10), les électrodes (20, 22) étant reliées à une source de courant électrique (130), la source de courant électrique fournissant un micro-courant mesurable, **caractérisé en ce que** les électrodes (20, 22) laissent le micro-courant mesurable s'écouler entre elles à travers la peau du patient ;
l'appareil étant en outre **caractérisé par** un dispositif d'alerte (120) associé auxdites électrodes (20, 22) et pour émettre un signal d'alerte à l'interruption dudit micro-courant s'écoulant entre lesdites électrodes (20, 22), ledit signal d'alerte étant configuré pourfermer une alimentation en fluide de ladite machine externe ou dudit dispositif de perfusion vers ledit dispositif d'accès intravasculaire (10) ; et
l'appareil étant en outre **caractérisé par** un dongle retenant ladite source de courant électrique (130) et ledit dispositif d'alerte (120).

2. Appareillage selon la revendication 1, dans lequel ledit dispositif d'alerte (120) est connecté pour fournir ledit signal d'alerte à ladite machine externe ou audit dispositif de perfusion.

3. Appareillage selon la revendication 1 ou 2, dans lequel ledit dispositif d'alerte (120) fournit ledit signal d'alerte à un émetteur pour fournir une alerte externe.

4. Appareillage selon la revendication 3, dans lequel ladite alerte externe est un membre du groupe consistant en une alerte tactile, une alerte audio, une alerte visuelle et une combinaison de celles-ci.

5. Appareillage selon la revendication 3, dans lequel ledit signal d'alerte est configuré pour notifier au patient ou à ses soignants une détection de déplacement.

6. Appareillage selon la revendication 3, dans lequel ladite alerte externe est à un emplacement distant.

7. Appareillage selon l'une quelconque des revendications précédentes, dans lequel le dispositif d'accès intravasculaire (10) comprend des ailettes pour retenir ladite aiguille sur la peau audit site d'accès et lesdites électrodes (20, 22) sont configurées pour être fixées sur les ailettes et à la peau au-delà des bords des ailettes.

8. Appareillage selon l'une quelconque des revendications précédentes, dans lequel le dispositif d'accès intravasculaire (10) est configuré pour être fixé sur la peau sur le site d'accès en utilisant de la bande adhésive et les électrodes (20, 22) sont configurées pour être fixées sur le site d'accès par rapport au dispositif d'accès intravasculaire (10).

9. Appareillage selon la revendication 8, dans lequel lesdites électrodes (20, 22) sont fixées à ladite bande adhésive.

10. Appareillage selon l'une quelconque des revendications précédentes, dans lequel lesdites électrodes (20, 22) sont fixées de manière indépendante sur le site d'accès par rapport au dispositif d'accès intravasculaire (10), ou dans lequel lesdites électrodes (20, 22) sont fixées audit dispositif d'accès intravasculaire (10) de façon à ce que le déplacement dudit dispositif d'accès intravasculaire provoque le déplacement d'une ou des deux électrodes (20, 22) de ladite peau.

11. Appareillage pour fournir une alerte de déplacement pour un dispositif d'accès intravasculaire (10) inséré dans un vaisseau sanguin d'un patient à travers la peau sur un site d'accès, l'appareillage comprenant :
des électrodes (20, 22) pour la fixation sur ladite peau à travers ledit site d'accès, les électrodes (20, 22) comprenant un laminé de plaquage de cuivre flexible (FCCL) ayant 3 couches : plaquage de cuivre, époxy et polyester ;
une source de courant électrique reliée auxdites électrodes (20, 22) ;
lesdites électrodes (20, 22) étant associées audit dispositif d'accès intravasculaire (10) de telle façon qu'au moins une desdites électrodes (20, 22) soit séparée de ladite peau au déplacement dudit dispositif d'accès intravasculaire (10), interrompant ainsi ledit micro-courant ;
un émetteur pour émettre un signal d'alarme à l'interruption du circuit de micro-courant et
un récepteur pour recevoir ledit signal d'alarme et pour produire une sortie d'alerte de déplacement, **caractérisé en ce que** :
lesdites électrodes sont configurées pour faciliter un micro-courant mesurable à travers ladite peau et entre lesdites électrodes (20, 22) à travers ledit site d'accès, la source de courant électrique étant située dans un dongle incluant un dispositif d'alerte (120),
la source de courant électrique fournissant ledit micro-courant mesurable auxdites électrodes, le micro-courant s'écoulant d'une première desdites électrodes (20, 22) à travers la peau du patient vers une seconde desdites électrodes (20, 22).

12. Appareillage selon la revendication 11, dans lequel ladite sortie d'alerte de déplacement est configurée pour faire fonctionner une machine de traitement de fluide traitant du fluide pris du, ou étant fourni au, dispositif d'accès intravasculaire (10) pour mettre la machine de traitement de fluide dans un état de sécurité, ou est configurée pour faire fonctionner une alarme à distance.
